# EUROPÄISCHE PATENTSCHRIFT

(11) **EP 0 971 948 B1**
(45) Veröffentlichungstag und Bekanntmachung des Hinweises auf die Patenterteilung: **13.08.2003**
(21) Anmeldenummer: 98919267.9
(22) Anmeldetag: 14.04.1998
(51) Int. Cl.: C07K 7/00

(54) **INHIBITOREN FÜR DEN UROKINASEREZEPTOR**
INHIBITORS FOR UROKINASE RECEPTOR
INHIBITEURS POUR LE RECEPTEUR DE L'UROKINASE

(30) Priorität: 11.04.1997 EP 97106024
(43) Veröffentlichungstag der Anmeldung: 19.01.2000
(73) Patentinhaber: Wilex AG, 81675 München (DE)
(72) Erfinder: KESSLER, Horst, D-65824 Schwalbach-Limes (DE); GRAEFF, Heinrich, D-81479 München (DE); SCHMITT, Manfred, D-81669 München (DE); MAGDOLEN, Viktor, D-85551 Kirchheim (DE); WILHELM, Olaf, G., D-81545 München (DE); RIEMER, Christoph, D-81677 München (DE); BÜRGLE, Markus, D-81369 München (DE)
(74) Vertreter: Weiss, Wolfgang, Dipl.-Chem. Dr.
(86) Internationale Anmeldenummer: EP9802179
(87) Internationale Veröffentlichungsnummer: WO98046632

(56) Entgegenhaltungen:
- WO-A-94/22464
- WO-A-95/14714
- V.MAGDOLEN ET AL.: "Systematic mutational analysis of the receptor- binding region of the human urokinase- type plasminogen activator" EUR.J.BIOCHEM., Bd. 237, 1996, Seiten 237-51, XP000652373 in der Anmeldung erwähnt
- I.M.TAKENAKA ET AL.: "Hsc70-binding Peptides Selected from a Phage Display Peptide Library that Resemble Organellar Targetting Sequences" J.BIOL.CHEM., Bd. 270, Nr. 34, 1995, Seiten 19839-44, XP002082849

## Beschreibung

Die vorliegende Erfindung betrifft Peptide als Inhibitoren der Bindung von Urokinase an den Urokinaserezeptor. Diese vorzugsweise zyklischen Peptide sind als pharmazeutische Wirkstoffe für Krankheiten geeignet, die durch Urokinase und ihren Rezeptor vermittelt werden.

Die Serinprotease uPA (Urokinase-Typ-Plasminogenaktivator) ist für verschiedene physiologische und pathologische Prozesse verantwortlich, wie etwa den proteolytischen Abbau von extrazellulärem Matrixmaterial, das für die Invasibilität und Wanderung von Zellen sowie für die Geweberemodellierung notwendig ist. uPA bindet mit hoher Affinität (K_{D}=10⁻¹⁰-10⁻⁹M) an den membranständigen uPA Rezeptor (uPAR) auf der Zelloberfläche.

Die Bindung von uPA an seinen Rezeptor ist an vielen invasiven biologischen Prozessen wie etwa der Metastasierung bösartiger Tumoren, der Trophoplastenimplantation, Entzündungen und Angiogenese beteiligt. Daher sind Antagonisten von uPA in der Lage, die Invasivität, Metastasierung und Angiogenese von Tumoren zu hemmen. uPA-Antagonisten können als Mittel zur Therapie invasiver und metastasierender Krebserkrankungen eingesetzt werden, bei denen uPA und uPAR an den invasiven Foci von Tumoren auftreten (Dano et al.: The receptor for urokinase plasminogen activator: Stromal cell involvement in extracellular proteolysis during cancer invasion, in: Proteolysis and Protein Turnover, Barrett, A. J. und Bond, J., HRSG, Portland Press, London, 1994, 239), z. B. bei Krebserkrankungen von Brust, Lunge, Darm und Ovarien. Darüber hinaus können uPA-Antagonisten auch für andere Zwecke eingesetzt werden, bei denen eine Hemmung der proteolytischen Aktivierung von Plasminogen erforderlich ist, beispielsweise zur Bekämpfung von Krankheiten wie Arthritis, Entzündungen, Osteoporose, Retinopathien und zur Kontrazeption.

Der uPA Rezeptor ist in WO 90/ 12091 sowie in den Veröffentlichungen Ploug et al., J. Biol. Chem. 268 (1993), 17539 und Ronne et al., J. Immunol. Methods 167 (1994), 91 beschrieben.

uPA wird als einzelkettiges Molekül (pro-uPA) synthetisiert und enzymatisch in einen aktiven zweikettigen uPA überführt. Das uPA Molekül besteht aus drei strukturell unabhängigen Domänen, der N-terminal lokalisierten wachstumsfaktorartigen Domäne (GFD, uPA 1 - 46), einer Kringelstrukturdomäne (uPA 45 - 135) und der Serinproteasedomäne (uPA 159 - 411). GFD und die Kringeldomäne bilden zusammen das sogenannte aminoterminale Fragment von uPA (ATF, uPA 1 - 135), das durch weitere proteolytische Spaltung von zweikettigem uPA erzeugt wird. ATF bindet an den uPA Rezeptor mit einer ähnlichen Affinität wie uPA.

Die rezeptorbindende Region von uPA erstreckt sich über den Bereich der Aminosäuren 12 bis 32, da ein Peptid, welches die Aminosäurereste 12 bis 32 von uPA enthält (wobei Cystein an Position 19 durch Alanin ersetzt ist) mit ATF um die Bindung an den uPA Rezeptor kompetiert (Appella et al., J. Biol. Chem. 262 (1987), 4437-4440). Weiterhin wurde dort gezeigt, daß dieses Peptid auch nach Zyklisierung durch Verbrückung der beiden Cysteinreste an den Positionen 12 und 32 eine Affinität für den uPA Rezeptor zeigt. In einem alternativen Ansatz wurden von Goodson et al., (Proc. Natl. Acad. USA 91 (1994), 7129-7133) antagonistische uPA-Peptide für den uPAR mittels Musterung einer Bakteriophagen-Peptidbibliothek identifiziert. Diese Peptide zeigten keine ersichtliche Sequenzhomologie zu der natürlichen uPAR-bindenden Sequenz von uPA.

In neueren Veröffentlichungen (Rettenberger et al., Biol. Chem. Hoppe-Seyler 376 (1995), 587-594); Magdolen et al., Eur. J. Biochem. 237 (1996), 743-751; Goretzki et al., Fibrinolysis and Proteolysis 11 (1997), 11-19) sind weitere Untersuchungen an der uPAR-Bindungsregion des uPA beschrieben. Dabei wurden die Reste Cys19, Lys23, Thyr24, Phe25, Ile28, Trp30 und Cys31 als wichtige Determinanten für eine uPA/uPAR-Wechselwirkung identifiziert. Als wirksamster Inhibitor wurde in diesen Untersuchungen ein uPA-Peptid mit den Aminosäuren 16 bis 32 von uPA identifiziert.

Magdolen et al. (1996) supra, analysieren die uPAR-Bindungsregion des uPA-Moleküls unter der Verwendung eines Peptids mit den Aminosäuren 14 bis 32 von uPA und davon abgeleiteten Peptiden. Diese sowie auch die von anderen Arbeitsgruppen verwendeten Peptide (vgl. z.B. Appella et al. (1987) supra) weisen jedoch eine relativ geringe Affinität zum uPAR auf.

WO 95/14714 stellt im Zusammenhang mit Untersuchungen an synthetischen, Integrin-bindenden Peptiden fest, dass das Vorliegen einer konformationsstabilisierten Konfiguration generell zu einer Erhöhung der Bindungsaffinität führt. Es wurde gezeigt, dass eine solche Stabilisierung z.B. durch zyklische Peptide beeinflusst sein kann.

Ein von Takenaka et al. (JBC 270 (34), (1995), 19839-19844) durchgeführter Vergleich der Bindung verschiedener Peptide an das Protein Hsc70 umfasst auch ein zyklisches Octapeptid und ein zyklisches Nona-Peptid. Diese wiesen gegenüber anderen Peptiden nur eine relativ schwache Affinität für dieses Protein auf.

WO-A-94/22464 offenbart lineare Peptide mit einer Länge von 6 bis 18 Aminosäuren, die aus dem Bereich der Aminosäuren 14 bis 33 von uPA stammen. Es wird beschrieben, daß kurze von uPA abgeleitete Peptide (uPA 21-29 und uPA 21-26) in der Lage sind, das Wachstum von Keratinozyten zu beeinflussen. Zwar wird in WO-A-94/22464 auf einen möglichen Einsatz der beanspruchten Peptide für die Blockierung der uPA/uPAR-Wechselwirkung hingewiesen, es werden jedoch keinerlei Daten oder Hinweise auf derartige Bindungsstudien gezeigt. Darüber hinaus enthalten die als bevorzugt genannten linearen Peptide uPA 21-29 und uPA 21-26 nicht die minimale uPAR-Bindungsregion von linearen uPA-Peptiden, welche den Sequenzbereich der Aminosäuren 19 bis 31 umfaßt. Die Beeinflussung des Wachstums von Keratinozyten durch diese kurzen Peptide beruht somit sehr wahrscheinlich nicht auf einer uPA/uPAR-Wechselwirkung.

Ein Nachteil der bisher bekannten uPA-Peptidinhibitoren besteht jedoch darin, daß sie eine relativ geringe Affinität der Bindung an den uPA Rezeptor zeigen, die für eine therapeutische Anwendung nicht ausreichend ist. Aufgrund dessen besteht ein großes Bedürfnis nach neuen uPA-Peptidantagonisten, die eine höhere Affinität für den Rezeptor aufweisen.

Überraschenderweise wurde in quantitativen Untersuchungen festgestellt, daß das lineare Peptid uPA (19-31), zyklische Derivate dieses Peptids und sequenzmodifizierte Peptide aus diesem uPA-Bereich eine deutlich verbesserte Affinität der Bindung an den uPA-Rezeptor zeigen.

Die Verwendbarkeit der erfindungsgemäßen Peptide als uPA-Antagonisten, die mit hoher Affinität an uPAR binden, wird durch experimentelle Daten belegt. Besonders bevorzugt sind dabei zyklische Peptide, die sich durch nicht im nativen uPA-Molekül vorkommende Verbrückungen, insbesondere Disulfidbrücken, auszeichnen.

Ein Gegenstand der vorliegenden Erfindung sind somit Peptide der allgemeinen Strukturformel (I):

X²¹-X²²-X²³-X²⁴-X²⁵-X²⁶-X²⁷-X²⁸-X²⁹-[X³⁰]ₙ - [Y]ₘ (I)

worin
X²¹ bis X³⁰ jeweils eine Aminocarbonsäure, vorzugsweise eine α-Aminocarbonsäure bedeuten und X²¹ und X²⁹ miteinander verbrückt sind,
Y ein Spacer ist,
m und n jeweils unabhängig 0 oder 1 sind,
und die monomeren Bausteine über -NR¹CO- oder -CONR¹- Bindungen verknüpft sind, wobei R¹ jeweils unabhängig Wasserstoff, Methyl oder Ethyl bedeutet, und pharmazeutisch verträgliche Salze und Derivate davon, und worin
die Aminocarbonsäurereste X²¹ bis X³⁰ jeweils unabhängig eine der folgenden Bedeutungen besitzen:
X²¹ und X²⁹ sind α-Aminocarbonsäurebausteine, die miteinander verbrückt werden können, besonders bevorzugt weisen sie eine SH-Seitenkette, insbesondere eine Cysteinseitenkette oder eine dazu strukturell verwandte Seitenkette, z.B. eine Penicillaminseitenkette, auf. Alternativ können X²¹ und X²⁹ auch zwei über eine Thioethergruppe verbundene α-Aminocarbonsäurereste sein, z.B. eine Lanthioningruppe.

X²² und X²⁷ sind jeweils unabhängig α-Aminocarbonsäuren mit einer aliphatischen Seitenkette, vorzugsweise einer aliphatischen hydrophilen Seitenkette, insbesondere einer Amidseitenkette wie etwa Asparagin oder Glutamin, insbesondere Asparagin.

X²³ ist eine α-Aminocarbonsäure mit einer basischen Seitenkette, z.B. Lysin, Ornithin oder Arginin oder mit einer aliphatischen hydrophilen Seitenkette, z.B. mit einer Amidseitenkette wie Glutamin oder Asparagin. Besonders bevorzugt ist X²³ Lysin.

X²⁴ und X²⁵ sind jeweils unabhängig α-Aminocarbonsäuren mit einer aromatischen Seitenkette, beispielsweise Tyrosin, Phenylalanin oder Tryptophan. Besonders bevorzugt ist X²⁴ Tyrosin und X²⁵ Phenylalanin.

X²⁶ ist eine α-Aminocarbonsäure mit einer aliphatischen Seitenkette, vorzugsweise mit einer aliphatischen hydrophilen Seitenkette wie etwa Hydroxyvalin, Homoserin, Serin oder Threonin, insbesondere Serin. X²⁶ kann aber auch eine aliphatische hydrophobe Seitenkette aufweisen wie etwa Alanin.

X²⁸ ist eine α-Aminocarbonsäure mit einer aliphatischen Seitenkette, vorzugsweise mit einer aliphatischen hydrophoben Seitenkette wie etwa Valin, Norvalin, Norleucin, Isoleucin, Leucin oder Alanin. Besonders bevorzugt ist X²⁸ Isoleucin.

X³⁰ - falls vorhanden - ist eine α-Aminocarbonsäure mit einer aromatischen Seitenkette, vorzugsweise mit einer Tryptophan-Seitenkette. Gegebenenfalls kann die Tryptophanseitenkette modifiziert sein, beispielsweise durch Reduktion.

Die erfindungsgemäßen Peptide sind vorzugsweise von der uPA-Sequenz abgeleitet und enthalten mindestens 2, besonders bevorzugt mindestens 3, beispielsweise 4 Aminosäurereste, die auch an entsprechenden Positionen der nativen uPA-Sequenz vorkommen. Besonders bevorzugt weisen mindestens 2 der Aminosäurereste X²², X²³, X²⁴, X²⁵, X²⁶, X²⁸ und X³⁰ eine gleiche Seitenkette wie eine Aminosäure an gleicher Position in der nativen uPA-Sequenz auf. Am meisten bevorzugt weisen mindestens 2 der Aminosäurereste X²⁴, X²⁵, X²⁸ und - falls vorhanden - X³⁰ die gleiche Seitenkette wie in der nativen uPA-Sequenz auf.

Y ist eine Spacergruppe, z. B eine aus einer oder mehreren Aminosäuren aufgebaute peptidische Spacergruppe, z.B. Poly-Lys, oder eine andere Spacergruppe, z.B. eine Polyethylenglykolgruppe. Das Peptid kann über die Gruppe Y an Trägersubstanzen gekoppelt werden.

Ein weiterer Gegenstand der vorliegenden Erfindung sind somit zyklische Peptide mit einem neungliedrigen Ring, wobei mindestens 2, vorzugsweise mindestens 3 und besonders bevorzugt mindestens 4 der den Ring bildenden Aminosäurereste eine Sequenz aus der uPA-Region 22 bis 28 aufweisen.

Neben Peptiden der Strukturformel (I) sind auch pharmazeutisch verträgliche Salze und Derivate davon als uPA-Antagonisten geeignet. Als Derivate kommen dabei insbesondere solche Verbindungen in Betracht, die an reaktiven Gruppen der Seitenkette oder/und des N- bzw. C-Terminus, z. B. an Amino- oder Carbonsäuregruppen, modifiziert sind. Beispiele für solche Modifikationen sind eine Acylierung, z. B. eine Acetylierung von Aminogruppen oder/und eine Amidierung oder Veresterung von Carbonsäuregruppen.

Als Bausteine für die erfindungsgemäßen Peptide verwendeten Aminocarbonsäuren können natürliche Aminosäuren bzw. deren Enantiomere oder auch nicht natürlich vorkommende Aminosäuren wie etwa γ-Aminobuttersäure, β-Alanin eingesetzt werden.

Die monomeren Bausteine sind über Säureamidbindungen NR¹CO oder CONR¹ verknüpft, d. h. die Peptidsequenzrichtung kann umgekehrt werden (Retropeptide). R¹ kann wie in nativen Polypeptiden Wasserstoff bedeuten. Andererseits kann R¹ jedoch auch einen Alkylrest, z. B. Methyl oder Ethyl, und insbesondere Methyl bedeuten, da durch eine N-Alkylierung der Amidbindung oftmals eine starke Aktivitätsbeeinflussung bewirkt werden kann (vgl. z. B. Levian-Teitelbaum et al., Biopolymers 28 (1989), 51-64).

Als monomere Bausteine können die α-Aminocarbonsäuren in Form von L-Enantiomeren oder/und D-Enantiomeren eingesetzt werden. Durch die Änderung der Chiralität kann die Raumstruktur der erfindungsgemäßen Peptide modifiziert werden, wodurch ebenfalls eine Aktivitätsbeeinflussung möglich wird. Besonders bevorzugt sind Retro-inverso Petide, d. h. Peptide, die in einer umgekehrten Sequenzrichtung vorliegen und D-Aminosäuren als monomere Bausteine enthalten. Bei diesen Retro-Inversostrukturen haben die funktionellen Seitenketten eine ähnliche räumliche Orientierung wie die in der nativen Peptidsequenz, sie können jedoch aufgrund des Vorhandenseins von D-Aminosäuren schlechter biologisch abgebaut werden und haben daher Vorteile als Arzneimittel (vgl. hierzu beispielsweise Wermuth et al., J. Am. Chem. Soc. 119 (1997), 1328-1335 und die darin genannten Zitate).

Die erfindungsgemäßen Peptide sind zyklische Verbindungen, wobei insbesondere die monomeren Bausteine X²¹ und X²⁹ miteinander verbrückt sind. Diese Verbrückung kann beispielsweise über die Seitenketten der jeweiligen α-Aminocarbonsäurereste erfolgen, wobei eine Verbrückung über Disulfidbindungen, z. B. zwischen zwei Cysteinresten besonders bevorzugt ist. Andere Arten der Zyklisierung zwischen Aminosäureseitenketten sind jedoch auch möglich, z. B. Amidbindungen zwischen einer Aminosäure mit einer Aminoseitengruppe, z. B. Ornithin oder Lys und einer Aminosäure mit einer Carbonsäureseitengruppe wie etwa Asp oder Glu. Weiterhin kann die Disulfidbrücke auch durch eine Alkylen-Brücke ersetzt werden, um die chemische Stabilität zu erhöhen. Darüber hinaus sind auch Verknüpfungen von einer Aminosäureseitenkette mit dem Peptidrückgrat, z. B. Verknüpfung einer Aminoseitengruppe, z.B. einer ω-Aminoseitengruppe, mit dem C-terminalen Ende bzw. Verknüpfung einer Carbonsäureseitengruppe mit dem N-terminalen Ende möglich. Auch eine Verknüpfung von N- und C-Terminus ist möglich.

Besonders bevorzugt ist, wenn mindestens eine der Aminosäuren X²¹, X²⁷, X²⁹ und X³⁰ eine D-Aminosäure ist. Besonders bevorzugt ist mindestens eine der Aminosäuren X²¹ und X³⁰ eine D-Aminosäure, z.B. D-Cystein.

Außerdem können anstelle der Disulfidbrücke auch sogenannte Turn-Mimetika (Haubner et al., J. Am. Chem. Soc. 118 (1996), 7884-7891) oder Zuckeraminosäuren (Graf von Rödern et al., J. Am. Chem. Soc. 118 (1996), 10156-10167) eingesetzt werden.

Die erfindungsgemäßen Peptide sind durch chemische Synthese wie in den Beispielen erläutert erhältlich. Alternativ können die erfindungsgemäßen Peptide auch Bestandteile von rekombinanten Polypeptiden sein.

Noch ein weiterer Gegenstand der vorliegenden Erfindung sind Peptide, die vom linearen Peptid uPA (19 bis 31) und zyklischen Derivaten davon abgeleitet sind und an ausgewählten Positionen D-Aminosäurereste tragen. Solche Peptide weisen die allgemeine Strukturformel (II) auf:

X¹-[X²]ₙ-[X³]ₘ-X⁴-K-Y-F-X⁵-X⁶-I-X⁷-W-[X⁸]ᵣ (II)

worin
- X¹ bis X⁸: jeweils eine Aminocarbonsäure, vorzugsweise eine α-Aminocarbonsäure bedeuten und X¹ und X⁷ oder X¹ und X⁸ miteinander verbrückt sind,
- n, m und r: jeweils unabhängig 0 oder 1 sind,
- K: wie X²³ definiert ist und vorzugsweise eine α-Aminocarbonsäure mit einer Lysin-Seitenkette bedeutet,
- Y: wie X³⁴ definiert ist und vorzugsweise eine α-Aminocarbonsäure mit einer Tyrosin-Seitenkette bedeutet,
- F: wie X²⁵ definiert ist und vorzugsweise eine α-Aminocarbonsäure mit einer Phenylalanin-Seitenkette bedeutet,
- I: wie X²⁸ definiert ist und vorzugsweise eine α-Aminocarbonsäure mit einer Isoleucin-Seitenkette bedeutet,
- W: wie X³⁰ definiert ist und vorzugsweise eine α-Aminocarbonsäure mit einer Tryptophan-Seitenkette bedeutet,
und die monomeren Bausteine über -CONR¹- oder -NR¹CO-Bindungen verknüpft sind, wobei R¹ jeweils unabhängig Wasserstoff, Methyl oder Ethyl bedeutet, und pharmazeutisch verträgliche Salze und Derivate davon und wobei mindestens einer der Aminosäurereste X¹, X², X³, X⁶, I, X⁷, W und X⁸ einen D-Aminosäurerest bedeutet.

Die monomeren Bausteine X¹ bis X⁸ weisen vorzugsweise folgende Bedeutungen auf:
X¹ und - falls vorhanden - X⁸ entsprechen der Bedeutung X²¹ und X²⁹ und sind z. B. α-Aminocarbonsäurebausteine mit einer SH-Seitenkette, insbesondere mit einer Cysteinseitenkette.
X² ist - falls vorhanden - eine α-Aminocarbonsäure mit einer aliphatischen und ungeladenen Seitenkette, z. B. Valin, Leucin oder Isoleucin, insbesondere Valin.
X³ und X⁵ entsprechen der Bedeutung von X²⁶ und sind z.B. α-Aminocarbonsäuren mit einer aliphatischen hydrophilen Seitenkette wie etwa Serin oder Threonin, insbesondere Serin.
X⁴ und X⁶ entsprechen der Bedeutung von X²² und X²⁷ und sind z.B. α-Aminocarbonsäuren mit einer aliphatischen hydrophilen Seitenkette, insbesondere einer Amidseitenkette wie etwa Asparagin oder Glutamin, insbesondere Asparagin.
X⁷ ist - wenn nicht mit X¹ verbrückt - vorzugsweise eine basische α-Aminocarbonsäure, insbesondere Histidin. Bei Verbrükkung mit X¹ ist X⁷ eine α-Aminocarbonsäure mit einer SH-Seitengruppe, insbesondere Cystein.

Weiterhin betrifft die vorliegende Erfindung eine pharmazeutische Zusammensetzung, die als Wirkstoff mindestens ein Peptid oder Polypeptid wie zuvor definiert gegebenenfalls zusammen mit pharmazeutisch üblichen Träger, Hilfs- oder Verdünnungsmitteln enthält. Insbesondere werden die erfindungsgemäßen Peptide oder Polypeptide zur Herstellung von uPA-Antagonisten verwendet, die sich auch zur Bekämpfung von mit der Expression von uPAR assoziierten Krankheiten, insbesondere zur Tumorbekämpfung eignen.

Ein weiterer Gegenstand der vorliegenden Erfindung ist die Verwendung von aus der uPA Sequenz abgeleiteten Peptiden, insbesondere von uPA Antagonisten wie etwa den zuvor genannten Peptiden oder Polypeptiden zur Herstellung von Targetingvehikeln, z.B. Liposomen, viralen Vektoren etc., für uPAR-exprimierende Zellen. Das Targeting kann für diagnostische Anwendungen zum gesteuerten Transport von Markierungsgruppen, z.B radioaktiven oder nichtradioaktiven Markierungsgruppen erfolgen. Andererseits kann das Targeting für therapeutische Anwendungen, z.B. zum Transport von pharmazeutischen Wirkstoffen, beispielsweise auch zum Transport von Nukleinsäuren für die Gentherapie erfolgen.

Die erfindungsgemäßen pharmazeutischen Zusammensetzungen können in beliebiger Form vorliegen, beispielsweise als Tabletten, als beschichtete Tabletten oder in Form von Lösungen oder Suspensionen in wässrigen oder nichtwässrigen Lösungsmitteln. Die Peptide werden vorzugsweise oral oder parenteral in einer flüssigen oder festen Form verabreicht. Bei Verabreichung in flüssiger Form wird Wasser vorzugsweise als Trägermedium verwendet, das gegebenenfalls Stabilisatoren, Löslichkeitsvermittler oder/und Puffer enthält, die üblicherweise für Injektionslösungen verwendet werden. Solche Zusatzstoffe sind beispielsweise Tartrat- oder Boratpuffer, Ethanol, Dimethylsulfoxid, Komplexierungsmittel wie etwa EDTA, Polymere wie etwa flüssiges Polyethylenoxid, etc.

Bei Verabreichung in fester Form können feste Trägersubstanzen wie etwa Stärke, Lactose, Mannitol, Methylcellulose, Talkum, hochdisperses Siliciumoxid, hochmolekulare Fettsäuren wie etwa Stearinsäure, Gelatine, Agar, Calciumphosphat, Magnesiumstearat, tierische und pflanzliche Fette oder feste hochmolekulare Polymere wie etwa Polyethylenglykole eingesetzt werden. Weiterhin können die Formulierungen zu oralen Applikation sofern gewünscht auch Aroma- und Süßstoffe enthalten.

Die erfindungsgemäßen therapeutischen Zusammensetzungen können auch in Form von Komplexen vorliegen, z.B. mit Cyclodextrinen wie etwa γ-Cyclodextrin.

Die verabreichte Dosis hängt vom Alter, Gesundheitszustand und Gewicht des Patienten, von der Art und der Schwere der Erkrankung, von der Art der Behandlung, von der Häufigkeit der Verabreichung und der Art der gewünschten Wirkung ab. Die tägliche Dosis der aktiven Verbindung ist üblicherweise 0,1 bis 50 mg/Kilogramm Körpergewicht. Normalerweise sind 0,5 bis 40 und vorzugsweise 1,0 bis 20 mg/kg/Tag in einer oder mehreren Dosen ausreichend, um die gewünschten Wirkungen zur erreichen.

Weiterhin soll die Erfindung durch die im Nachfolgenden beschriebenen Beispiele und Figuren erläutert werden.

Es zeigen:
- Figur 1: die mengenabhängige Hemmung der Bindung von pro-uPA an einen zelloberflächenassoziierten uPAR durch synthetische Peptide;
- Figur 2: die Kompetition synthetischer Peptide mit ATF um die Bindung an uPAR;
- Figur 3A: die Struktur von cyclo¹⁹⁻³¹ uPA 19-31 (rechts) im Vergleich zur Struktur der entsprechenden Domäne aus nativem uPA;
- Figur 3B: die Struktur des zyklischen Peptidderivats cyclo^{21,29} [Cys 21,29]uPA₂₁₋₃₀;
- Figur 4: die Hemmung der uPA/uPAR-Interaktion mittels synthetischer Peptide und
- Figur 5: die Hemmung des Tumorwachstums in Nacktmäusen durch Verabreichung synthetischer Peptide.

### Beispiele

### 1. Methoden

### 1.1 Festphasenpeptidsynthese

Lineare Peptide wurden auf einem 2-Chlortritylharz (Barlos et al., Int. J. Pept. Protein Res. 37 (1991), 513 bis 520) unter Verwendung eines Applied Biosystems Modell 431 A Peptidsynthesizers bzw. eines multiplen Peptidsynthesizers Modell Syro II (MultiSynTech) synthetisiert. Unter Anwendung der orthogonalen Fmoc-Strategie (Carpino und Han, J. Org. Chem. 37 (1972), 3404-3409; Fields und Noble, Int. J. Peptide Protein Res. 35 (1990), 161-214) wurden die Aminosäureseitenketten mit den Schutzgruppen Trityl (Asn, Cys, Gln und His), tert.-Butyloxycarbonyl (Lys und Trp), tert.-Butyl (Asp, Glu, Ser, Thr und Tyr) , Acetamidomethyl (Cys) und 2,2,5,7,8-Pentamethylchroman-6-sulfonyl oder 2,2,4,6,7-Pentamethyldihydrobenzofuran-5-sulfonyl (Arg) blockiert. Die Kupplung erfolgte bei Raumtemperatur in Dimethylformamid unter Verwendung eines dreifachen Überschusses von 2-(1H-Benzotriazol-1-yl)-1,1,3,3-tetramethyluroniumtetrafluoroborat/1-Hydroxybenzotriazol/Fmoc-Aminosäure mit 2,5 Äquivalenten von N-Ethyldiisopropylamin in N-Methylpyrrolidon. Die Fmoc-Gruppe wurde durch sequenzielle Behandlung der Harze mit einem Überschuß von 40 % bzw. 20 % Piperidin in Dimethylformamid entfernt. Die Abspaltung der Peptide und die Entfernung der Seitenkettenschutzgruppen wurde gleichzeitig durch Behandlung mit 82,5 % Trifluoressigsäure/5 % Phenol/2,5 % Ethandithiol/5 % Thioanisol/5 % H₂O (0 °C/1 h; Raumtemperatur/1h) durchgeführt. Im Fall von mit 2,2,5,5,7,8-Pentamethylchroman-6-sulfonyl-geschützten Arg-Gruppen wurden die Peptide für zusätzliche 12 h bei Raumtemperatur inkubiert. Die rohen Peptide wurden mit Diethylether bei -30 °C präzipitiert, in Methanol gelöst, wie zuvor präzipitiert, in tert.-Butanol gelöst und lyophilisiert. Tryptophan enthaltende Peptide wurden zusätzlich mit 5 % Essigsäure für 2 h vor der Lyophilisierung behandelt.

Die Peptide wurden durch HPLC unter Verwendung einer Reverse Phase C-18 Säule (Nucleosil 1005-C18) oder einer YMC-Pack ODS Säule gereinigt. Die Zyklisierung erfolgte durch eine Disulfidbrückenbildung über die Cysteinreste. Die hierzu erforderliche Oxidation wurde durch Aufnehmen von 0,1 bis 0,3 mg/ml der gereinigten linearen Peptide in 80 % Wasser und 20 % DMSO (Vol/Vol) und Entfernen des Lösungsmittels unter verringertem Druck nach 10 h durchgeführt. Die zyklischen Peptide wurden erneut durch HPLC wie zuvor beschrieben gereinigt.

### 1.2 Massenspektroskopie und Aminosäureanalyse

Die gereinigten und entsalzten Peptide wurden auf dem HPLC-System 140 B (Applied Biosystems, Foster City, USA) analysiert. Die UV-Absorption wurde mit einem UVIS 200 Detektor (Linear Instruments, Reno, USA) bei 206 nm gemessen. Die Chromatographie erfolgte auf einer Aquapore 3 µ (Applied Biosystems, Foster City, USA) Reverse Phase Säule (1 mm x 50 mm) mit einer Durchflußrate von 20 µl/min. Das Lösungsmittelsystem war 0,1 % TFA in Wasser (A) und 0,1 % TFA in Acetonitril (B). Das HPLC System war an eine Atmosphärendruck-Ionisationsquelle gekoppelt, die an ein Tandem-Quadrupolinstrument API III (Sciex, Perkin Elmer, Thornhill, Kanada) angeschlossen war.

Die Quadrupol M/Z-Skala wurde mit den Ammoniumadduktionen von Polypropylenglykol kalibriert. Die durchschnittlichen Massenwerte wurden von den M/Z-Peaks in den Ladungsverteilungsprofilen der mehrfach geladenen Ionen berechnet (Covey et al., Rapid Commun. Mass Spectrom. 2 (1988), 249-256; Fenn et al., Science 246 (1989), 64-71).

Die Aminosäureanalyse wurde nach der Ninhydrinmethode unter Verwendung des Analysesystems 6300 (Beckman Instruments, Fullerton, USA) nach Hydrolyse der Peptide durch die TFA-HCl-Dampfphasenmethode (Tsugita et al., J. Biochem. 102 (1987), 1593-1597) durchgeführt, die eine quantitative Bestimmung der Peptidkonzentration erlaubt.

### 1.3 Durchflußzytometrie

Die Kapazität der synthetischen Peptide zu Hemmung der uPA/uPAR-Wechselwirkung wurde mittels Durchflußzytometrie an dem FACScan Durchflußzytometer (Becton-Dickinson, Heidelberg, Deutschland) unter Verwendung der humanen Promyeloidzellinie U937 als Quelle für zellulären nativen uPAR bestimmt (Chuchulowski et al., Fibrinolysis 6, Suppl. 4 (1992), 95-102; Magdolen et al., (1996), supra). Die U937 Zellen wurden mit 1 mM Phorbol-12-myristat-13-acetat (PMA) für 48 h stimuliert. Nach Stimulierung mit PMA exprimieren die U937 Zellen beträchtliche Mengen an zelloberflächenassoziiertem uPAR.

Die stimulierten Zellen wurden mit 50 mM Glycin-HCl, 0,1 NaCl, pH 3,6 für 1 min bei Raumtemperatur behandelt, um endogenen rezeptorgebundenen uPA zu dissoziieren. Anschließend wurde der saure Puffer durch 0,5 M HEPES-100 mM NaCl, pH 7,5 neutralisiert. Die Zellen wurden dann sofort zweimal mit PBS/0,1 % Rinderserumalbumin (RSA) gewaschen und für 10 min bei Raumtemperatur und 300 x g zentrifugiert. Die Zellen wurden in PBS/0,1 % RSA resuspendiert, auf eine Konzentration von 10⁶ Zellen pro ml eingestellt und simultan mit 16 ng FITC-konjugiertem pro-uPA und unterschiedlichen Mengen der synthetischen Peptide für 45 min bei Raumtemperatur inkubiert. Vor der Analyse wurde Propidiumiodid, ein Fluoreszenzfarbstoff, der spezifisch doppelsträngige DNA bindet, zu jeder Probe gegeben, um die Lebensfähigkeit der analysierten U937 Zellen zu bestimmen. Die beschädigten, Propidiumiodid-markierten Zellen wurden von der Analyse ausgeschlossen.

### 1.4 Festphasen uPAR/uPA-Bindetest

Zusätzlich zu den durchflußzytometrischen Analysen wurde ein Festphasen ATF-Ligandenbindetest zur Untersuchung der Wechselwirkungen von synthetischen Peptiden mit uPAR durchgeführt. Hierzu wurden Mikrotiterplatten mit rekombinantem humanem uPAR aus CHO Zellen (Wilhelm et al., FEBS Lett. 337 (1994), 131-134; Magdolen et al., Elektrophoresis 16 (1995), 813-816) beschichtet und die restlichen proteinbindenden Stellen mit 2 % RSA (Gew/Vol) abgesättigt. Nach Inkubation mit den Proben (0,6 ng ATF zusammen mit 15 µg synthetisches Peptid pro ml) und mehrfachen Waschschritten wurde die Menge an ATF, die an den auf der Mikrotiterplatte immobilisierten uPAR gebunden hatte, unter Verwendung eines biotinylierten monoklonalen Mausantikörpers gegen die Kringeldomäne von ATF (Nr. 377, American Diagnostica, Greenwich, CT, USA) und anschließender Zugabe von Avidin-Peroxidase-Konjugat und 3,3',5,5'-Tetramethylbenzidin/H₂O₂ als Substrat für die Peroxidase bestimmt. Das Vorhandensein von synthetischen Peptiden, die mit der ATF-Bindung an uPAR kompetieren, verringert den Umsatz des chromogenen Substrats.

### 2. Ergebnisse

### 2.1 Bestimmung der uPAR Bindungskapazität von synthetischen Peptiden durch quantitative durchflußzytometrische Analyse

Es wurde die inhibitorische Kapazität der Peptide uPA₁₂₋₃₂[C19A] (Appella et al., (1987), supra), des sogenannten Klon 20-Peptids AEPMPHSLNFSQYLWYT (Goodson et al., (1994), supra), welches das wirksamste aus einer Phagen Peptidbibliothek identifzierte Peptid war, und des von der Wildtyp uPA-Sequenz abgeleiteten synthetischen Peptid uPA₁₆₋₃₂ verglichen.

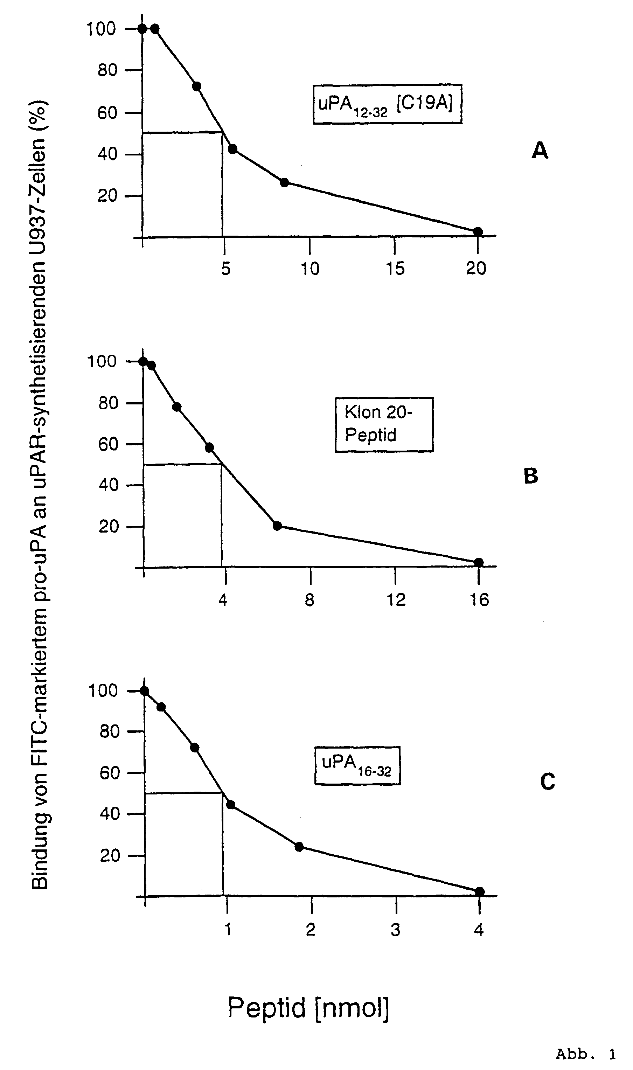

Hierzu wurden die gereinigten Peptide durch Massenspektroskopie analysiert, durch Aminosäureanalyse quantifiziert und dann gemäß der in 1.3 beschriebenen Methode durchflußzytometrisch auf ihre Fähigkeit zur Inhibierung der Bindung von fluoreszenzmarkiertem pro-uPA an den uPA Rezeptor auf U937 Zellen getestet. Dabei wurde gefunden, daß pro-uPA von zelloberflächenassoziiertem uPAR durch alle drei synthetischen Peptide in einer dosisabhängigen Weise verdrängt wurde (Fig.1). Ein etwa 15.000 bzw. 12.000 molarer Überschuß an uPA₁₂₋₃₂[C19A] bzw. Klon 20 Peptid führte zu einer 50 %igen Hemmung der Bindung von uPA. Das Peptid uPA₁₆₋₃₂ zeigte eine 4- bis 5-fach höhere Affinität an uPAR verglichen mit den zwei anderen Peptiden: Ein etwa 3.000-facher molarer Überschuß reichte aus, um eine 50 %ige Hemmung zu erhalten.

Weiterhin wurde festgestellt, daß das lineare Peptid uPA₁₉₋₃₁ überraschenderweise einen IC50-Wert von ca. 0,8 µM zeigt, während der IC50-Wert für uPA₁₆₋₃₂ ca. 3,2 µM beträgt.

### 2.2 Bestimmung der uPAR-Bindekapazität von synthetischen Peptiden in einem Mikrotiterplatten-Festphase-Liganden-Bindetest.

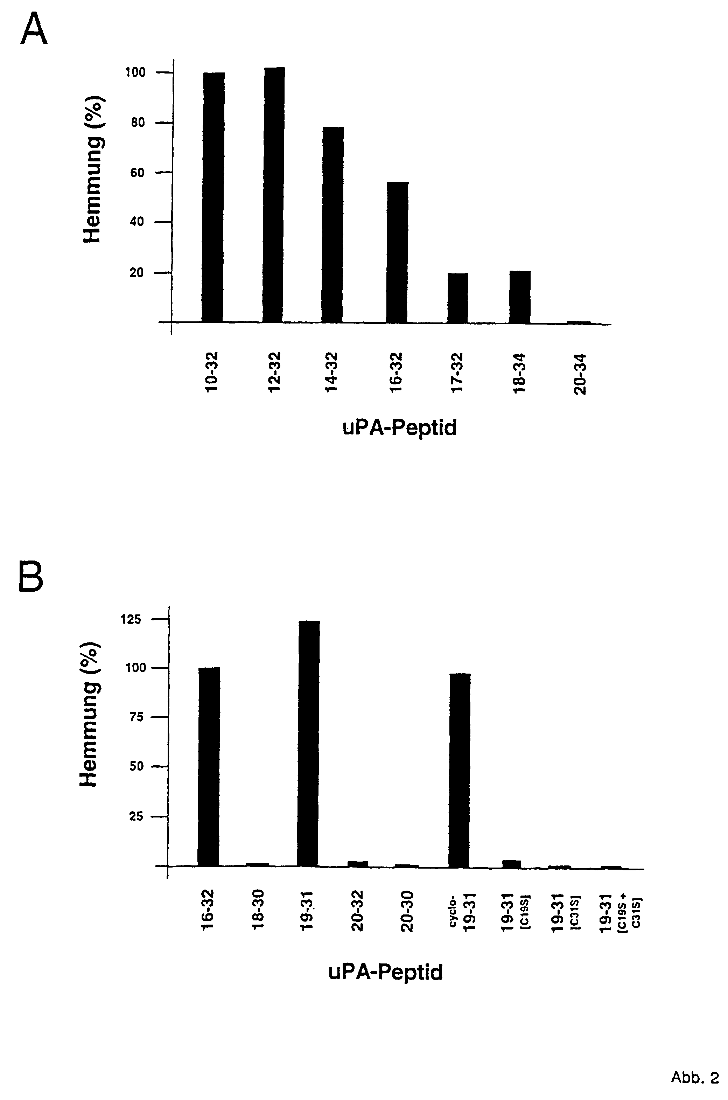

Es wurde eine Reihe von Peptiden mit variablen Sequenzbereichen aus dem rezeptorbindenden Bereich von uPA synthetisiert, die ausgehend von uPA₁₀₋₃₂ zunehmend vom Aminoterminus verkürzt wurden. Zur Bestimmung der inhibitorischen Kapazität dieser Peptide wurde der unter 1.4 beschriebene Mikrotiterplatten-Festphasenbindetest verwendet. Die Ergebnisse dieses Tests sind in Fig. 2 dargestellt.

Aus Fig. 2A ist ersichtlich, daß die Peptide uPA₁₀₋₃₂, uPA₁₂₋₃₁, uPA₁₄₋₃₂ und uPA₁₆₋₃₂ wirksam die Bindung von ATF an uPAR inhibieren. Die Peptide uPA₁₇₋₃₂ und uPA₁₈₋₃₄ zeigten deutlich verringerte uPAR-Bindekapazitäten. Das Peptid uPA₂₀₋₃₄ zeigte überhaupt keine Bindung an den uPAR. In einem weiteren Versuch wurde die Bindekapazität der Peptide uPA₁₉₋₃₁, uPA₁₈₋₃₀, uPA₂₀₋₃₂ und uPA₂₀₋₃₀ getestet. Das Ergebnis dieses Versuchs ist in Fig. 2B dargestellt. Überraschenderweise wurde festgestellt, daß uPA₁₉₋₃₁ mit höherer Affinität als das längere Peptid uPA₁₆₋₃₂ an den uPAR bindet. Die anderen getesteten linearen Peptide zeigten keine signifikante Bindekapazität.

Das zyklische Peptid cyclo¹⁹⁻³¹uPA₁₉₋₃₁, welches eine intramolekulare Disulfidbindung zwischen den Cysteinresten an den Positionen 19-31 enthält, war überraschenderweise immer noch in der Lage, die Bindung von uPA an den uPA Rezeptor zu hemmen. cyclo¹⁹⁻³¹uPA₁₉₋₃₁ war darüber hinaus in seiner Bindeaktivität signifikant stabiler nach längerer Aufbewahrung in wässriger Lösung oder wiederholten Einfrier/Tau-Zyklen verglichen mit dem linearen Peptid uPA₁₉₋₃₁.

### 2.3 Systematisches Ersetzen von L-Aminosäuren durch D-Aminosäuren bei chemisch synthetisierten linearen und zyklischen Peptiden aus dem Bereich uPA₁₉₋₃₁

Es wurde die uPAR-Bindungskapazität von synthetischen linearen und zyklischen Peptiden aus dem Bereich uPA₁₉₋₃₁ bestimmt, bei denen jeweils eine L-Aminosäure durch die entsprechende D-Aminosäure ersetzt worden war. Die Ergebnisse dieses Experiments sind in der nachfolgenden Tabelle 1 gezeigt.

**Tabelle 1**

| **D-Aminosäure** | **Peptidstruktur** | **Hemmung** |
|---|---|---|
| Trp30 | [D-Trp³⁰]uPA₁₉₋₃₁ | ++ |
| Trp30 | cyclo[D-Trp³⁰]uPA₁₉₋₃₁ | + |
| His29 | [D-His²⁹]uPA₁₉₋₃₁ | ++ |
| His29 | cyclo[D-His²⁹]uPA₁₉₋₃₁ | + |
| Asn27 | [D-Asn²⁷]uPA₁₉₋₃₁ | ++ |
| Asn27 | cyclo [D-Asn²⁷]uPA₁₉₋₃₁ | ++ |
| Ser21 | [D-Ser²¹]uPA₁₉₋₃₁ | ++ |
| Ser21 | cyclo[D-Ser²¹]uPA₁₉₋₃₁ | ++ |
| Val20 | [D-Val²⁰]uPA₁₉₋₃₁ | ++ |
| Val20 | cyclo[D-Val²⁰]uPA₁₉₋₃₁ | + |
| Cysl9 | [D-Cys¹⁹]uPA₁₉₋₃₁ | +++ |
| Cysl9 | cyclo[D-Cys¹⁹]uPA₁₉₋₃₁ | +++ |
| cyclol9-31 | cyclo[19-31]uPA₁₉₋₃₁ | +++ |

Aus dieser Tabelle-ist ersichtlich, daß die Einführung von D-Aminosäuren an den Positionen Cys19, Va120, Ser21, Asn27, His29 und Trp30 sowohl bei linearen als auch zyklischen Peptiden möglich ist, ohne daß die Hemmwirkung verlorengeht. Darüber hinaus wurde festgestellt, daß bei linearen Peptiden die Hemmwirkung auch bei Einführen von D-Aminosäuren an den Positionen Ile28 und Cys31 nicht verlorengeht.

### 2.4 Synthese von modifizierten zyklischen uPA Peptiden

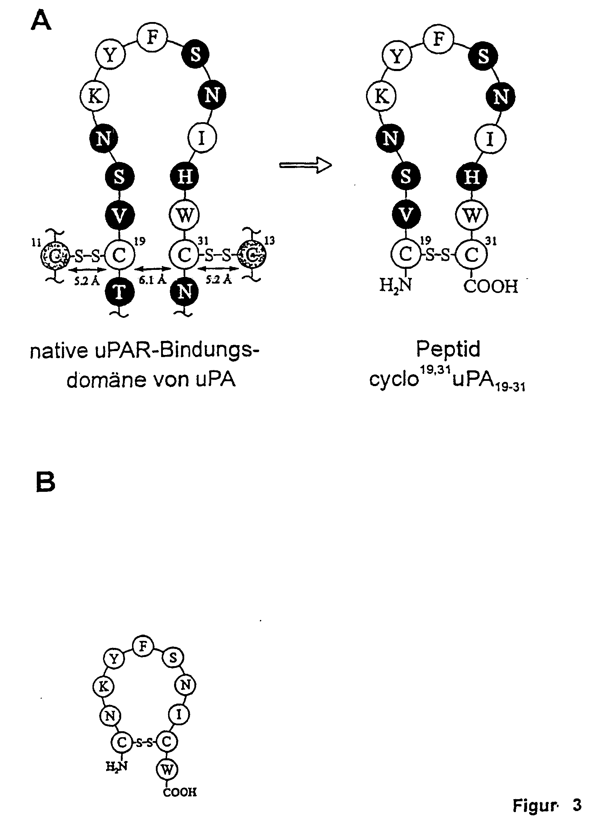

Unter Verwendung von cyclo^{19,31}uPA₁₉₋₃₁ als Leitstruktur wurde ein zyklisches Peptid hergestellt, in dem bestimmte Aminosäuren deletiert und/oder durch andere Aminosäuren substituiert waren. Die Struktur dieser neuen synthetischen Peptidvariante cyclo^{21,29}[Cys21,29]uPA₂₁₋₃₀ ist in Fig. 3 gezeigt. In Abweichung von der unter 1.1 angegebenen Synthesemethode erfolgt die Herstellung dieses Peptids an einem Tritylchlorid-Polystyrolharz.

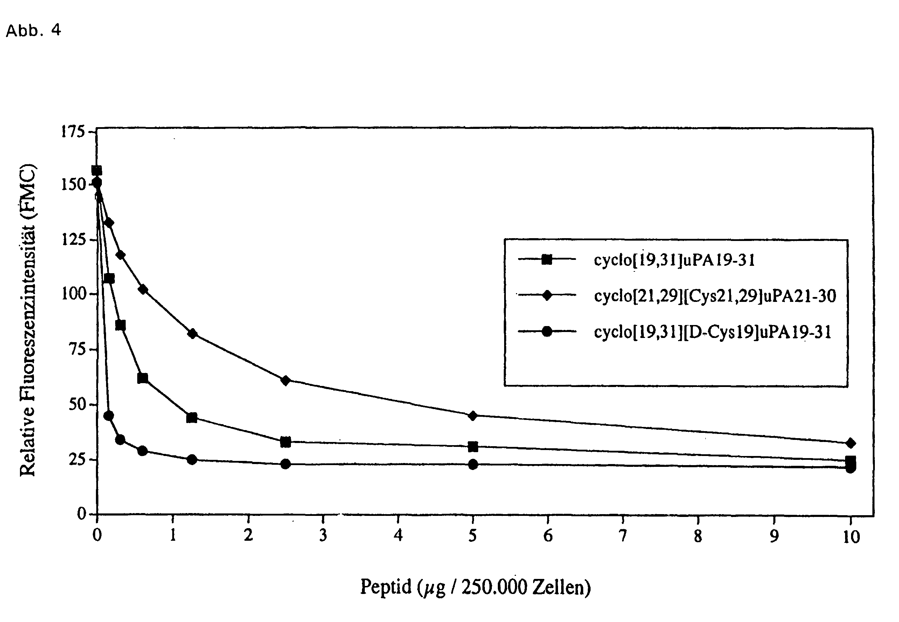

Fig. 4 zeigt die inhibitorische Wirkung dieser synthetischen Peptidvariante im Vergleich zu cyclo^{19.31}uPA₁₉₋₃₁ und cyclo^{19,31}[D-Cys19]uPA₁₉₋₃₁.

### 2.5 in vivo Wirkung

4-6 Wochen alten Balbc/3 Nacktmäusen wurden an der rechten Flanke 6x10⁶ humane Brustkrebszellen MDA-MB-231 (Price et al., Cancer Res. 50 (1990), 717-721) in einem Gesamtvolumen von 300 µl injiziert. Vor der Injektion wurden die Krebszellen mit jeweils 200 µg der zyklischen uPA-Peptide cyclo^{19,31}uPA₁₉₋₃₁ und cyclo^{21,29}[Cys21,29]uPA₂₁₋₃₀ in PBS pH 7,4 vermischt. Anschließend wurden die Mäuse zweimal wöchentlich mit dem jeweiligen Peptid in einer Dosis von 10 mg/kg Körpergewicht intraperitonial behandelt (Injektionsvolumen 300 µl). Das Volumen der in den Mäusen auftretenden Primärtumoren in cm³ wurde nach 1, 2, 3, und 5 Wochen durch Messung an den beiden größten Durchmessern bestimmt. Den Kontrollmäusen wurde PBS pH 7,4 verabreicht. Jede Gruppe bestand aus 5 Mäusen. Die Ergebnisse für das Peptid cyclo^{19,31}uPA₁₉₋₃₁ sind in Tab. 2 gezeigt.

**Tabelle 2**

| Woche | Kontrolle | uPA-Peptid |
|---|---|---|
| 1 | 0 | 0 |
| 2 | 0,34 ± 0,3 | 0,086 ± 0,047 |
| 3 | 0,71 ± 0,5 | 0,303 ± 0,129 |
| 5 | 2,33 ± 0,32 | 0,62 ± 0,21* |

| | | |
|---|---|---|
| *p=0,02 | | |

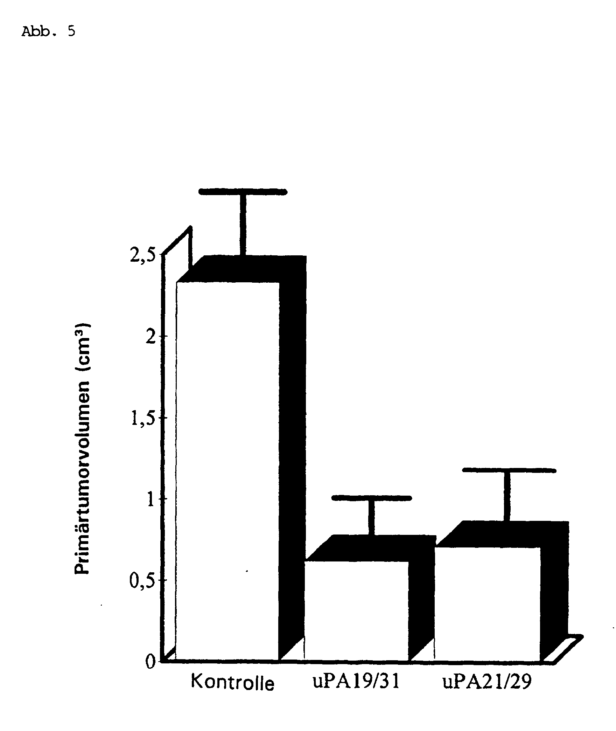

In Fig. 5 ist das Volumen des Primärtumors nach fünfwöchiger Behandlung gezeigt. Es ist zu erkennen, daß die Verabreichung beider Peptide zu einer signifikanten Verringerung des Tumorwachstums in vivo führte.

## Patentansprüche

1. Peptide der allgemeinen strukturformel (I):
X²¹-X²²-X²³-X²⁴-X²⁵-X²⁶-X²⁷-X²⁸-X²⁹-[X³⁰]ₙ - [Y]ₘ (I)
worin
X¹¹ bis X³⁰ jeweils eine Aminocarbonsäure bedeuten und X²¹ und X²⁹ miteinander verbrückt sind,
Y ein Spacer ist,
n und m jeweils unabhängig 0 oder 1 sind,
und die monomeren Bausteine über -NR¹CO- oder -CONR¹-Bindungen verknüpft sind, wobei R¹ jeweils unabhängig Wasserstoff, Methyl oder Ethyl bedeutet, und pharmazeutisch verträgliche Salze und Derivate davon und worin die Aminosäurereste X²¹-X³⁰ jeweils unabhängig eine der nachfolgend angegebenen Bedeutungen besitzen:
(i) X²¹ und X²⁹ sind jeweils Aminocarbonsäurereste mit einer SH-Seitenkette oder 2 Aminocarbonsäurereste, die über eine Thioetherbindung verbrückt sind;
(ii) X²² und X²⁷ sind jeweils unabhängig Aminocarbonsäurereste mit einer aliphatischen Seitenkette, vorzugsweise mit einer aliphatischen hydrophilen Seitenkette, besonders bevorzugt mit einer Amidseitenkette;
(iii) X²³ ist ein Aminocarbonsäurerest mit einer basischen oder einer aliphatischen hydrophilen Seitenkette;
(iv) X²⁴, X²⁵ und X³⁰ sind jeweils unabhängig Aminocarbonsäurereste mit aromatischen Seitenketten,
(v) X²⁶ ist ein Aminocarbonsäurerest mit einer aliphatischen Seitenkette, vorzugsweise mit einer aliphatischen hydrophilen Seitenkette besonders bevorzugt mit einer Hydroxyseitenkette und
(vi) X²⁸ ist eine Aminocarbonsäurerest mit einer aliphatischen Seitenkette, vorzugsweise mit einer aliphatischen hydrophoben Seitenkette.

2. Verbindungen nach Anspruch 1,
**dadurch gekennzeichnet,**
**daß** X²⁴, X²⁵, X²⁸ und X³⁰ jeweils unabhängig eine der nachfolgenden Bedeutungen besitzen:
(i) X²⁴ hat eine Tyrosin-Seitenkette;
(ii) X²⁵ hat eine Phenylalanin-Seitenkette;
(iii) X²⁸ hat eine Alanin-, Leucin- oder Isoleucin-Seitenkette, vorzugsweise eine Isoleucin-Seitenkette und
(iv) X³⁰ hat eine gegebenenfalls modifizierte Tryptophan-Seitenkette.

3. Verbindungen nach einem der vorhergehenden Ansprüche,
**dadurch gekennzeichnet,**
**daß** mindestens 2 der Aminosäurereste X²², X²³, X²⁴, X²⁵, X²⁶, X²⁷, X²⁸ und X³⁰ eine gleiche Seitenkette wie eine Aminosäure an gleicher Position in der nativen uPA-Sequenz aufweisen.

4. Verbindungen nach Anspruch 3,
**dadurch gekennzeichnet,**
**daß** mindestens 2 der Aminosäurereste X²⁴, X²⁵, X²⁸ und X³⁰ die gleiche Seitenkette wie in der nativen uPA-Sequenz aufweisen.

5. Verbindungen nach einem der vorhergehenden Ansprüche,
**dadurch gekennzeichnet,**
**daß** die Verbrückung von X²¹ und X²⁹ über Seitenketten der Aminocarbonsäurereste erfolgt.

6. Verbindungen nach Anspruch 5,
**dadurch gekennzeichnet,**
**daß** die Verbrückung über Disulfidbindungen erfolgt.

7. Verwendung von zyklischen Peptide mit einem neungliedrigen Ring zur Herstellung eines uPA-Antagonisten oder eines Targetinvehikels für uPAR-exprimierende Zellen,
**dadurch gekennzeichnet,**
**daß** mindestens 2 der den Ring bildenden Aminosäurereste eine aus dem Bereich der Aminosäuren 22-28 von uPA stammende Sequenz aufweisen.

8. Verwendung nach Anspruch 7,
**dadurch gekennzeichnet,**
**daß** die den Ring bildenden Aminosäurereste eine Bedeutung entsprechend den Aminoresten X²¹ bis X²⁹ gemäß den Ansprüchen 1 bis 7 besitzen.

9. Polypeptide,
**dadurch gekennzeichnet,**
**daß** sie als Strukturelemente mindestens ein Peptid nach einem der Ansprüche 1 bis 6 enthalten.

10. Peptide der allgemeinen Strukturformel (II):
X¹- [X²]ₙ- [X³]ₘ-X⁴-K-Y-F-X⁵-X⁶-I-X⁷-W-[X⁸]ᵣ (II)
worin:
X¹, X², X³, X⁴, X⁵, X⁶, X⁷ und X⁸ jeweils eine Aminocarbonsäure bedeuten und die monomeren Bausteine X¹ und X⁷ oder X¹ und X⁸ miteinander verbrückt sind,
n, m und r jeweils unabhängig 0 oder 1 sind,
K eine α-Aminocarbonsäure mit einer Lysin-Seitenkette bedeutet,
Y eine α-Aminocarbonsäure mit einer Tyrosin-Seitenkette bedeutet,
F eine α-Aminocarbonsäure mit einer Phenylalanin-Seitenkette bedeutet,
I eine α-Aminocarbonsäure mit einer Isoleucin-Seitenkette bedeutet,
W eine α-Aminocarbonsäure mit einer Tryptophan-Seitenkette bedeutet,
und die monomeren Bausteine über -NR¹CO- oder -CONR¹-Bindungen verknüpft sind, wobei R¹ jeweils unabhängig Wasserstoff, Methyl oder Ethyl bedeutet, und pharmazeutisch verträgliche Salze und Derivate davon,
**dadurch gekennzeichnet,**
**dass** mindestens einer der Aminosäurereste X¹, X², X³, X⁶, I, X⁷, W und X⁸ einen D-Aminosäurerest bedeutet.

11. Pharmazeutische Zusammensetzung, die als Wirkstoff mindestens ein Peptid oder Polypeptid nach einem der Ansprüche 1 bis 6 oder 9 bis 10 gegebenenfalls zusammen mit pharmazeutisch üblichen Träger-, Hilfs- oder Verdünnungsmitteln enthält.

12. Verwendung eines Peptids oder Polypeptids nach einem der Ansprüche 1 bis 6 oder 9 bis 10 zur Herstellung eines uPA-Antagonisten.

13. Verwendung
(i) eines Peptids oder Polypeptids nach einem der Ansprüche 1-6 oder 9-10 oder eines aus diesem Peptid oder Polypeptid hergestellten uPA-Antagonisten; oder
(ii) eines Peptids oder Polypeptids mit einem neungliedrigen Ring, bei dem mindestens 2 der den Ring bildenden Aminosäurereste eine aus dem Bereich der Aminosäuren 22 bis 28 von uPA stammende Sequenz aufweisen und bei dem die den Ring bildenden Aminosäurereste gegebenfalls eine Bedeutung entsprechend der Aminosäurereste X²¹ bis X²⁹ gemäß den Ansprüchen 1 bis 7 besitzen, oder eines aus diesem Peptid oder Polypeptid hergestellten uPA-Antagonisten oder Targetingvehikels für uPAR exprimierende Zellen
zur Herstellung eines Arzneimittels zur Bekämpfung von mit der Expression von uPAR-assoziierten Krankheiten, insbesondere zur Tumorbekämpfung.

14. Verwendung eines Peptids oder Polypeptids nach einem der Ansprüche 1 bis 6 oder 9 bis 10 zur Herstellung eines Targetingvehikels für uPAR-exprimierende Zellen.

## Claims

1. Peptides of the general structural formula (I):
X²¹-X²²-X²³-X²⁴-X²⁵-X²⁶-X²⁷-X²⁸-X²⁹-[X³⁰]ₙ - [Y]ₘ (I)
in which
X²¹ to X³⁰ each mean an aminocarboxylic acid and X²¹ and
X²⁹ are bridged together,
Y is a spacer,
n and m are each independently 0 or 1,
and the monomeric building blocks are linked by -NR¹-CO- or -CONR¹- bonds, R¹ in each case independently denoting hydrogen, methyl or ethyl, and pharmaceutically compatible salts and derivatives thereof and in which the amino acid residues X²¹-X³⁰ each independently have one of the following meanings:
(i) X²¹ and X²⁹ are each aminocarboxylic acid residues with an SH side chain or 2 aminocarboxylic acid residues which are bridged by a thioether bond;
(ii) X²² and X²⁷ are each independently aminocarboxylic acid residues with an aliphatic side chain, preferably with an aliphatic hydrophilic side chain, particularly preferably with an amide side chain;
(iii) X²³ is an aminocarboxylic acid residue with a basic or an aliphatic hydrophilic side chain;
(iv) X²⁴, X²⁵ and X³⁰ are each independently aminocarboxylic acid residues with aromatic side chains,
(v) X²⁶ is an aminocarboxylic acid residue with an aliphatic side chain, preferably with an aliphatic hydrophilic side chain, particularly preferably with a hydroxy side chain and
(vi) X²⁸ is an aminocarboxylic acid residue with an aliphatic side chain, preferably with an aliphatic hydrophobic side chain.

2. Compounds according to claim 1,
**characterised in that**
X²⁴, X²⁵, X²⁸ and X³⁰ each independently have one of the following meanings:
(i) X²⁴ has a tyrosine side chain;
(ii) X²⁵ has a phenylalanine side chain;
(iii) X²⁸ has an alanine, leucine or isoleucine side chain, preferably an isoleucine side chain and
(iv) X³⁰ has an optionally modified tryptophan side chain.

3. Compounds according to one of the previous claims,
**characterised in that**
at least 2 of the amino acid residues X²², X²³, X²⁴, X²⁵, X²⁶, X²⁷, X²⁸ and X³⁰ have an identical side chain to an amino acid at the same position in the native uPA sequence.

4. Compounds according to claim 3,
**characterised in that**
at least 2 of the amino acid residues X²⁴, X²⁵, X²⁸ and X³⁰ have the same side chain as in the native uPA sequence.

5. Compounds according to one of the previous claims,
**characterised in that**
X²¹ and X²⁹ are bridged via side chains of the aminocarboxylic acid residues.

6. Compounds according to claim 5,
**characterised in that**
they are bridged by means of disulfide bonds.

7. Use of cyclic peptides with a nine-membered ring to produce a uPA antagonist or a targeting vehicle for uPAR-expressing cells,
**characterised in that**
at least 2 of the amino acid residues forming the ring comprise a sequence originating from the region of amino acids 22-28 of uPA.

8. Use according to claim 7,
**characterised in that**
the amino acid residues forming the ring have a meaning corresponding to amino residues X²¹ to X²⁹ according to claims 1 to 7.

9. Polypeptides,
**characterised in that**
they contain at least one peptide according to one of claims 1 to 6 as a structural element.

10. Peptides of the general structural formula (II):
X¹-[X²]ₙ-[X³]ₘ-X⁴-K-Y-F-X⁵-X⁶-I-X⁷-W-[X⁸]ᵣ (II)
in which
X¹, X², X³, X⁴, X⁵, X⁶, X⁷ and X⁸ each mean an aminocarboxylic acid and the monomeric building blocks X¹ and X⁷ or X¹ and X⁸ are bridged together,
n, m and r are each independently 0 or 1,
K means an α-aminocarboxylic acid with a lysine side chain,
Y means an α-aminocarboxylic acid with a tyrosine side chain,
F means an α-aminocarboxylic acid with a phenylalanine side chain,
I means an α-aminocarboxylic acid with an isoleucine side chain,
W means an α-aminocarboxylic acid with a tryptophan side chain,
and the monomeric building blocks are linked by -NR¹CO- or -CONR¹- bonds, R¹ in each case independently denoting hydrogen, methyl or ethyl, and pharmaceutically compatible salts and derivatives thereof,
**characterised in that**
at least one of the amino acid residues X¹, X², X³, X⁶, I, X⁷, W and X⁸ means a D-amino acid residue.

11. A pharmaceutical composition which contains at least one peptide or polypeptide according to one of claims 1 to 6 or 9 to 10 as an active substance, optionally together with conventional pharmaceutical excipients, auxiliary agents or diluents.

12. Use of a peptide or polypeptide according to one of claims 1 to 6 or 9 to 10 to produce a uPA antagonist.

13. Use
(i) of a peptide or polypeptide according to one of claims 1-6 or 9-10 or of a uPA antagonist produced from said peptide or polypeptide; or
(ii) of a peptide or polypeptide with a nine-membered ring, in which at least 2 of the amino acid residues forming the ring comprise a sequence originating from the region of amino acids 22-28 of uPA and in which the amino acid residues forming the ring optionally have a meaning corresponding to amino residues X²¹ to X²⁹ according to claims 1 to 7, or of a uPA antagonist produced from said peptide or polypeptide or of a targeting vehicle for uPAR-expressing cells
to produce a pharmaceutical to combat diseases associated with the expression of uPAR, in particular to combat tumours.

14. Use of a peptide or polypeptide according to one of claims 1 to 6 or 9 to 10 to produce a targeting vehicle for uPAR-expressing cells.

## Revendications

1. Peptides de formule développée générale (I)
X²¹-X²²-X²³-X²⁴-X²⁵-X²⁶-X²⁷-X²⁸-X²⁹-[X³⁰]ₙ-[Y]ₘ (I)
dans laquelle
les X²¹ à X³⁰ représentent chacun un acide aminocarboxylique et X²¹ et X²⁹ sont liés entre eux par un pont,
Y est un espaceur,
n et m sont chacun indépendamment 0 ou 1,
et les éléments constitutifs monomères sont liés par l'intermédiaire de liaisons -NR¹CO- ou -CONR¹-, où R¹ représente à chaque fois indépendamment un atome d'hydrogène ou un groupe méthyle ou éthyle, et leurs sels et dérivés acceptables sur le plan pharmaceutique, les restes d'aminoacides X²¹ à X³⁰ ayant chacun indépendamment l'une des significations indiquées ci-dessous:
(i) X²¹ et X²⁹ sont chacun des restes d'acides aminocarboxyliques ayant une chaîne latérale SH ou représentent deux restes d'acides aminocarboxyliques pontés par l'intermédiaire d'une liaison thioéther;
(ii) X²² et X²⁷ sont chacun indépendamment des restes d'acides aminocarboxyliques ayant une chaîne latérale aliphatique, de préférence une chaîne latérale aliphatique hydrophile, de façon particulièrement préférée une chaîne latérale amide;
(iii) X²³ est un reste d'acide aminocarboxylique ayant une chaîne latérale basique ou une chaîne latérale aliphatique hydrophile;
(iv) X²⁴, X²⁵ et X³⁰ sont chacun indépendamment des restes d'acides aminocarboxyliques ayant des chaînes latérales aromatiques;
(v) X²⁶ est un reste d'acide aminocarboxylique ayant une chaîne latérale aliphatique, de préférence une chaîne latérale aliphatique hydrophile, de façon particulièrement préférée une chaîne latérale hydroxy, et
(vi) X²⁸ est un reste d'acide aminocarboxylique ayant une chaîne latérale aliphatique, de préférence une chaîne latérale aliphatique hydrophobe.

2. Composés selon la revendication 1, **caractérisés en ce que** X²⁴, X²⁵, X²⁸ et X³⁰ ont chacun indépendamment l'une des significations suivantes:
(i) X²⁴ a une chaîne latérale de tyrosine;
(ii) X²⁵ a une chaîne latérale de phénylalanine;
(iii) X²⁸ a une chaîne latérale d'alanine, de leucine ou d'isoleucine, de préférence une chaîne latérale d'isoleucine; et
(iv) X³⁰ a une chaîne latérale de tryptophane éventuellement modifiée.

3. Composés selon l'une des revendications précédentes, **caractérisés en ce qu'**au moins deux des restes d'aminoacides X²², X²³, X²⁴, X²⁵, X²⁶, X²⁷, X²⁸ et X³⁰ ont la même chaîne latérale que l'aminoacide à la même position dans la séquence de l'uPA natif.

4. Composés selon la revendication 3, **caractérisés en ce qu'**au moins deux des restes d'aminoacides X²⁴, X²⁵, X²⁸ et X³⁰ ont la même chaîne latérale que dans la séquence de l'uPA natif.

5. Composés selon l'une des revendications précédentes, **caractérisés en ce que** le pontage de X²¹ et X²⁹ s'effectue par l'intermédiaire de chaînes latérales des restes d'acides aminocarboxyliques.

6. Composés selon la revendication 5, **caractérisés en ce que** le pontage s'effectue par l'intermédiaire de liaisons disulfure.

7. Utilisation de peptides cycliques ayant un cycle de neuf chaînons pour la préparation d'un antagoniste de l'uPA ou d'un véhicule de ciblage pour des cellules exprimant l'uPAR, **caractérisée en ce qu'**au moins deux des restes d'aminoacides formant le cycle présentent une séquence provenant du domaine des aminoacides 22-28 de l'uPA.

8. Utilisation selon la revendication 7, **caractérisée en ce que** les restes d'aminoacides formant le cycle ont une signification correspondant aux restes d'aminoacides X²¹ à X²⁹ selon les revendications 1 à 7.

9. Polypeptides, **caractérisés en ce qu'**ils contiennent comme éléments de structure au moins un peptide selon l'une des revendications 1 à 6.

10. Peptides de formule développée générale (II):
X¹-[X²]ₙ-[X³]ₘ-X⁴-K-Y-F-X⁵-X₆-I-X⁷-W-[X⁸]ᵣ (II)
dans laquelle
X¹, X², X³, X⁴, X⁵, X⁶, X⁷ et X⁸ représentent chacun un acide aminocarboxylique et les éléments constitutifs monomères X¹ et X⁷ ou X¹ et X⁸ sont reliés entre eux par un pont,
n, m et r sont indépendamment les uns des autres 0 ou 1,
K représente un acide α-aminocarboxylique avec une chaîne latérale de lysine,
Y représente un acide α-aminocarboxylique avec une chaîne latérale de tyrosine,
F représente un acide α-aminocarboxylique avec une chaîne latérale de phénylalanine,
I représente un acide α-aminocarboxylique avec une chaîne latérale d'isoleucine,
W représente un acide α-aminocarboxylique avec une chaîne latérale de tryptophane,
et les éléments constitutifs monomères sont liés par l'intermédiaire de liaisons -NR¹CO- ou -CONR¹-, où R¹ représente à chaque fois indépendamment un atome d'hydrogène ou un groupe méthyle ou éthyle, et leurs sels et dérivés acceptables sur le plan pharmaceutique, **caractérisés en ce qu'**au moins l'un des restes d'aminoacides X¹, X², X³, X⁶, I, X⁷, W et X⁸ représente un reste de D-aminoacide.

11. Composition pharmaceutique contenant comme substance active au moins un peptide ou polypeptide selon l'une des revendications 1 à 6 ou 9 à 10, éventuellement avec des supports, des agents auxiliaires ou des diluants classiques en pharmacie.

12. Utilisation d'un peptide ou d'un polypeptide selon l'une des revendications 1 à 6 ou 9 à 10 pour la préparation d'un antagoniste de l'uPA.

13. Utilisation
(i) d'un peptide ou polypeptide selon l'une des revendications 1 à 6 ou 9 à 10 ou d'un antagoniste de l'uPA préparé à partir de ce peptide ou polypeptide; ou
(ii) d'un peptide ou polypeptide contenant un cycle de neuf chaînons dans lequel au moins deux des restes d'aminoacides formant le cycle présentent une séquence provenant du domaine des aminoacides 22 à 28 de l'uPA et dans lequel les restes d'aminoacides formant le cycle ont éventuellement une signification correspondant aux restes d'aminoacides X²¹ à X²⁹ selon les revendications 1 à 7, ou d'un antagoniste de l'uPA ou d'un véhicule de ciblage pour des cellules exprimant l'uPAR préparé à partir de ce peptide ou polypeptide,
pour la préparation d'un médicament destiné à la lutte contre les maladies associées à l'expression de l'uPAR, en particulier pour la lutte contre les tumeurs.

14. Utilisation d'un peptide ou polypeptide selon l'une des revendications 1 à 6 ou 9 à 10 pour la préparation d'un véhicule de ciblage pour des cellules exprimant l'uPAR.
